# EUROPEAN PATENT APPLICATION

(11) **EP 1 530 953 A1**
(43) Date of publication of application: **18.05.2005**
(21) Application number: 04257104.2
(22) Date of filing: 16.11.2004
(51) Int. Cl.: A61C 3/03, A61C 3/00

(54) **Periodontal scaling instruments**

(30) Priority: 17.11.2003 GB 0326724; 27.04.2004 GB 0409358
(71) Applicant: Pring, Matthew James, Sheffield S11 7GZ (GB)
(72) Inventor: Pring, Matthew James, Sheffield S11 7GZ (GB)
(74) Representative: Atkinson, Ralph

(57) **Abstract**

A curette or like instrument for use by a dental practitioner in removing plaque and calculus from tooth surfaces within periodontal pockets comprises a tip **10** having a debridement portion **16**. The debridement portion **16** is provided with a marking in the form of mutually contrasting bands **16a, 16b, 16c** and **16d** to give the practitioner a visual indication of the extent to which the instrument has in use penetrated a periodontal pocket, so that its end **14** (which is sharp) does not cause pain or injury to the patient. The bands **16a, 16b, 16c** and **16d** are formed, eg by etching or by differently coloured materials, to accord with markings already used on periodontal probes. The invention is applicable to ultrasonic, sonic and piezo-electric scaling instruments as well as to manual instruments.

## Description

This invention concerns periodontal scaling instruments used by dental practitioners for removing plaque and calculus from tooth surfaces within periodontal pockets.

A periodontal pocket is a space between a tooth and the adjacent gum and the removal of plaque and calculus from a tooth surface within such a pocket generally requires the professional attention of a qualified dental surgeon or hygienist. According to need, the practitioner may use any one or more of a variety of scaling instruments, generally known as scalers (which term is herein deemed to include sickles, hoes and jaquette scalers) and curettes.

The depth of a periodontal pocket - that is, the amount by which it extends inward of the gingival margin - is a diagnostic of oral health. As such it is commonly measured by a dental practitioner, and several different probes are available for this purpose, including those known as CPITN probes, BPE probes and Weston probes. These all bear marking to show the depth of the pocket when the tip of the probe engages the inward end of the pocket. A typical probe has marking in the form of contrasting bands showing depths of 3.5mm, 5.5mm, 8.5mm and 11.5mm.

It may be noted at this point that, although periodontal probes have been in widespread use for many years, it has never previously been thought useful to apply similar marking to a periodontal scaling instrument. Heretofore, even when a pocket depth has been measured, a practitioner has had to rely on skill, experience and dexterity to know how far down to extend a scaling instrument: too little, and plaque or calculus may be left to accumulate, with damaging consequences for the patient's oral health; too far, and the patient will be hurt and possibly injured.

It is an object of the present invention to help a dental practitioner to know how far to penetrate a periodontal pocket when removing plaque or calculus from a tooth surface therewithin.

Thus according to the invention there is provided a periodontal scaling instrument having a tip formed for removing plaque and/or calculus from a tooth surface within a periodontal pocket, characterised in that said tip has marking to indicate, in use, its extent of penetration into the pocket.

The marking may comprise mutually contrasting bands or a plurality of grooves.

Many periodontal scaling instruments are used with the tip at an inclination to the tooth surface, and to accommodate this the tip marking may be inclined relative to the tip at an angle substantially equal to the inclination of the tip on the tooth surface in use. Further, practitioners often use instruments such as sickles and curettes in such a way that the inclination is varied during use, and to accommodate this the marking may vary along said tip to correspond to a varying inclination of the tip in use.

The marking may be provided by paint or the like applied to said tip or it may be formed in the tip, eg by etching, by laser etching or by machining.

Certain different materials of use in periodontal scaling instruments differ in colour. For instance, hard titanium and tungsten alloys used for the cutting edges of periodontal scaling instruments are commonly quite different in appearance from the steel shafts of such instruments. Such different materials may be utilised to provide the instrument marking.

The marking may replicate marking of a CPITN probe or a BPE probe or a Weston probe, or it may have some other marking.

The instrument may be configured and arranged for manual use or for attachment to a sonic, ultrasonic or piezo-electric scaling unit.

The invention will now be described by way of example only with reference to the accompanying drawings, in which -
*Figure 1* shows enlarged in side elevation a tip of a periodontal curette embodying the invention;
*Figure 2* shows enlarged in side elevation a tip of a periodontal hoe embodying the invention;
*Figure 3* shows enlarged in side elevation a tip of a periodontal jaquette scaler embodying the invention;
*Figure 4* shows enlarged in side elevation a tip of a periodontal sickle embodying the invention;
*Figure 5* shows further enlarged in plan view the tip of the periodontal sickle shown in *Figure 4;* and
*Figure 6* illustrates the application of the invention to an ultrasonic scaler.

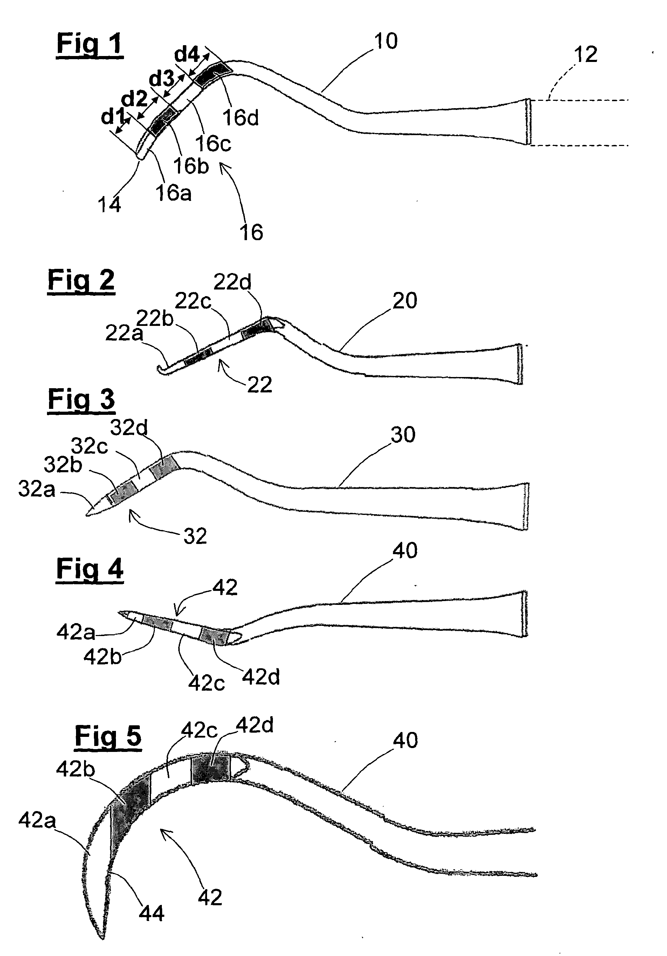

Referring first to *Figure 1*, this shows a tip **10** of a curette used for removing plaque and calculus from tooth surfaces within periodontal pockets. As shown in *Figure 1* the tip **10** is attached to a handle, indicated partially in broken lines at **12.** The way in which the curette is used will be readily understood by those skilled in the art, and for present purposes it will be sufficient to say that its free end **14** is entered into a periodontal pocket and the adjacent debridement portion **16** is drawn over the tooth surface within the pocket to remove plaque and/or calculus.

As can be seen in *Figure 1,* the debridement portion **16** has a marking in the form of mutually contrasting bands **16a, 16b, 16c** and **16d.** The bands **16b** and **16d** are etched, to contrast with the bands **16a** and **16c**, which are left plain. The bands **16a**, **16b**, **16c** and **16d** together provide a visual indication of the extent to which the debridement portion **16** has penetrated the periodontal pocket, so that its end **14** (which is sharp) does not cause pain or injury to the patient.

To make it easy for the practitioner to understand the indication, the bands **16a**, **16b**, **16c** and **16d** are formed in accordance with the marking of a CIPTN periodontal probe. Thus the band **16a** has a width d1 (its dimension from the end **14**) wherein d1 = 3.5mm; and the band **16b** has a width d2 wherein d2 = 3.0mm, the band **16c** has a width d3 wherein d3 = 2.5mm and the band **16d** has a width d4 wherein d4 = 3.0mm. However it is to be understood that the bands may be dimensioned in any way appropriate to provide a visual indication of the extent to which the tip penetrates a periodontal pocket.

*Figure 2* shows a tip **20** of a hoe having a debridement portion **22** marked with mutually contrasting bands **22a, 22b, 22c** and **22d** to indicate the extent of its penetration into a periodontal pocket.

*Figure 3* shows a tip **30** of a jaquette scaler having a debridement portion **32** marked with mutually contrasting bands **32a, 32b, 32c** and **32d** to indicate the extent of its penetration into a periodontal pocket.

*Figure 4* shows a tip **40** of a sickle having a debridement portion **42** marked with mutually contrasting bands **42a, 42b, 42c** and **42d** to indicate the extent of its penetration into a periodontal pocket.

The debridement portion or "blade" of a sickle (and of some other periodontal scaling instruments) is arcuate and thus its inclination or "angle of attack" on a tooth surface varies along its length. It follows that any marking to indicate depth of penetration into a periodontal pocket must be similarly inclined. This is illustrated by *Figure 5,* from which can be seen that the bands **42a, 42b, 42c** and **42d** are inclined relative to the cutting edge **44** and that the angle of inclination varies along the (arcuate) length of the debridement portion **42.**

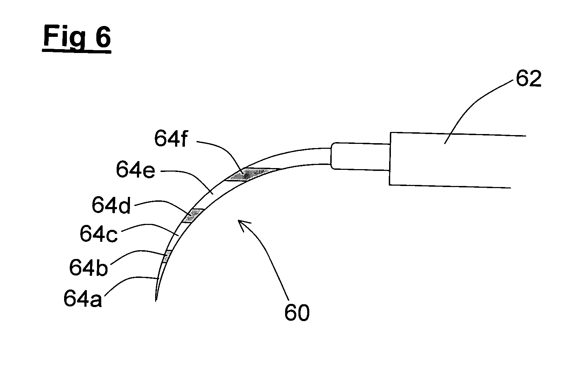

Referring now to *Figure* 6, this shows a tip **60** used for ultrasonic removal of plaque or calculus within a periodontal pocket. To this end the tip **60** is caused to vibrate by an ultrasonic scaling unit, not shown, and drawn over a tooth surface by means of a handle **62.** (It should be noted that a similar tip may be used with a sonic or a piezo-electric scaling unit). The tip **60** is marked with contrasting bands **64a**, **64b**, **64c**, **64d**, **64e** and **64f**.

Various modifications may be made to the instruments described without departing from the scope of the invention. For instance, for sickles and other instruments which are commonly rotated somewhat during use, so that the angle of attack is altered, the marking may be curved or otherwise formed to indicate depth of penetration for different degrees of rotation. It will also be understood that the marking may be provided by means other than etching, for instance by paint or the like. Other modifications will be apparent to those skilled in the art.

## Claims

1. A periodontal scaling instrument having a tip (**10**, **20**, **30**, **40**) formed for removing plaque and/or calculus from a tooth surface within a periodontal pocket, **characterised in that** said tip has marking to indicate, in use, its extent of penetration into the pocket.

2. A periodontal scaling instrument as claimed in claim **1 characterised in that** said marking comprises mutually contrasting bands (**16a**, **16b**, **16c**, **16d** etc).

3. A periodontal scaling instrument as claimed in claim **1 characterised in that** said marking comprises a plurality of grooves.

4. A periodontal scaling instrument as claimed in any preceding claim **characterised in that** said marking (**42a**, **42b**, **42c** and **42d**) is inclined relative to the tip (**40**) at an angle substantially equal to the inclination of the tip on the tooth surface in use.

5. A periodontal scaling instrument as claimed in claim **4 characterised in that** said marking (**42a**, **42b**, **42c** and **42d**) varies along said tip (**40**) to correspond to a varying inclination of the tip in use.

6. A periodontal scaling instrument as claimed in any preceding claim **characterised in that** the marking is provided by paint or the like applied to said tip.

7. A periodontal scaling instrument as claimed in any of claims **1** to **5 characterised in that** the marking is formed in the tip.

8. A periodontal scaling instrument as claimed in claim **7 characterised in that** the marking is formed by etching.

9. A periodontal scaling instrument as claimed in claim **7 characterised in that** the marking is formed by laser etching.

10. A periodontal scaling instrument as claimed in claim **7 characterised in that** the marking is formed by machining.

11. A periodontal scaling as claimed in any preceding claim, **characterised in that** said tip is formed of materials of different colours whereby said marking is provided.

12. A periodontal scaling instrument as claimed in claim **11 characterised in that** said materials are different metals.

13. A periodontal scaling instrument as claimed in any preceding claim **characterised in that** the marking replicates marking of a CIPTN probe or a BPE probe or a Weston probe.

14. A periodontal scaling instrument as claimed in any preceding claim, **characterised in that** said instrument is configured and arranged for manual use.

15. A periodontal scaling instrument as claimed in any of claims **1** to **14**, **characterised in that** said instrument (**60**) is configured and arranged for attachment to a sonic, ultrasonic or piezo-electric scaling unit.

16. A periodontal scaling instrument substantially as hereinbefore described with reference to and as shown in the accompanying drawings.
